**Europäisches Patentamt**

**European Patent Office**

**Office européen des brevets**

(19)

(11) Veröffentlichungsnummer: **0 124 755**
**B1**

(12) # EUROPÄISCHE PATENTSCHRIFT

(45) Veröffentlichungstag der Patentschrift:
16.07.86

(51) Int. Cl.⁴: **C 07 C 121/413**, C 07 C 120/00

(21) Anmeldenummer: 84103541.3

(22) Anmeldetag: 30.03.84

(54) Verfahren zur Herstellung von 2-Cyanacrylaten aus 2,4-Dicyanglutaraten.

(30) Priorität: 07.04.83 DE 3312427

(43) Veröffentlichungstag der Anmeldung:
14.11.84 Patentblatt 84/46

(45) Bekanntmachung des Hinweises auf die Patenterteilung:
16.07.86 Patentblatt 86/29

(84) Benannte Vertragsstaaten:
AT BE CH DE FR GB IT LI LU NL SE

(56) Entgegenhaltungen:
US - A - 2 926 188
US - A - 3 254 111

(73) Patentinhaber: **Henkel Kommanditgesellschaft auf Aktien, Postfach 1100 Henkelstrasse 67, D-4000 Düsseldorf-Holthausen (DE)**

(72) Erfinder: **Harth, Hubert, Dr., Buchenstrasse 31, D-4000 Düsseldorf-13 (DE)**
Erfinder: **Wüst, Willi, Dr., Fasanenring 32, D-4030 Ratingen 6 (DE)**
Erfinder: **Bruhn, Athanas, Dr., Haupstrasse 57, D-8870 Günzburg (DE)**
Erfinder: **Danielisz, Miklos, Dr., Girlitzpark 31, D-3000 Hannover 61 (DE)**
Erfinder: **Heinrich, Erbo, Dr., Bröhnweg 11, D-3015 Wennigsen (DE)**

**Beschreibung**

Die Erfindung liegt auf dem Gebiet der Monomerklebstoffe, sie betrifft ein neues Verfahren zur Herstellung von monomeren 1-Cyanacrylaten.

2,4-Dicyanglutarate (symmetrische Diester der 2,4-Dicyanglutarsäure) sind durch Umsetzung von 2 mol Cyanacetat mit 1 mol Formaldehyd herstellbare Rohstoffe für die 2-Cyanacrylatsynthese, entstehen aber auch als Nebenprodukte bei der Herstellung von 2-Cyanacrylaten. Es hat daher zahlreiche Bemühungen gegeben, diese wertvollen Rohstoffe – seien sie nun gezielt hergestellt oder als Rückstand bei der thermischen Spaltung von Oligomeren 2-Cyanacrylaten im Gemenge mit anderen Substanzen erhalten – in 2-Cyanacrylate zu überführen.

So wird in der US-PS 2 926 188 vorgeschlagen, 2,4-Dicyanglutarate thermisch in 2-Cyanacrylate und Cyanacetate zu spalten. Das Verfahren arbeitet bei Temperaturen von 175°C bis zu 210°C. Diese hohen Reaktionstemperaturen führen jedoch zu Nebenreaktionen und damit zur Ausbeuteminderung. Es wurde deshalb in der älteren deutschen Patentanmeldung DE-A-3 233 007 der Anmelderin vorgeschlagen, die Thermolyse durch Verwendung von Basen wie Alkali- bzw. Erdalkalialkoholate zu katalysieren. Dadurch wird es möglich, die Reaktion bei Temperaturen zwischen 90 und 130°C durchzuführen und 2-Cyanacrylate in erhöhter Ausbeute zu erhalten. Da sich in Abhängigkeit von dem zur Veresterung verwendeten Alkohol die Siedepunkte von Cyanacetaten und 2-Cyanacrylaten teilweise nur geringfügig unterscheiden, sind bei beiden genannten Verfahren teilweise aufwendige Destillationsvorrichtungen nötig, um die Produkte in geforderter Reinheit herstellen zu können.

Weiterhin ist es aus der US-PS 3 254 111 bekannt, 2,4-Dicyanglutarate mit Paraformaldehyd in Gegenwart basischer Katalysatoren zu kondensieren und das so erhaltene Produkt thermisch zu 2-Cyanacrylaten zu spalten. Bei dieser Vorgehensweise sind die relativ geringen Ausbeuten an 2-Cyanacrylat nachteilig. Verwendet man Amine, z. B. Piperidin als Kondensationskatalysatoren, so werden überdies Produkte mit schlechten Klebeeigenschaften erhalten. Weiterhin erfordert die Entfernung des als Schleppmittel verwendeten Benzols einen Zusatzaufwand.

Es besteht somit Bedarf nach einem neuen Verfahren zur Herstellung von 2-Cyanacrylaten aus 2,4-Dicyanglutaraten bei relativ niederen Temperaturen in guten Ausbeuten. Fernerhin sollten die Produkte die für Verklebungen geforderte Reinheit aufweisen.

Aufgabe der Erfindung ist es somit, ein Verfahren zur Herstellung von 2-Cyanacrylaten aus 2,4-Dicyanglutaraten bereitzustellen, das diesen Anforderungen genügt. Eine weitere Aufgabe der Erfindung betrifft die Verbesserung der Ausbeute bei der Herstellung von 2-Cyanacrylaten aus Cyanacetaten und Formaldehyd bzw. Polyoxymethylen durch verbessertes Recycling der als Nebenprodukte anfallenden 2,4-Dicyanglutarate.

Gegenstand der Erfindung ist somit ein Verfahren zur Herstellung von monomeren 2-Cyanacrylaten durch Umsetzung von 2,4-Dicyanglutaraten mit Formaldehyd und/oder Polyoxymethylen und anschliessender thermischer Spaltung der entstandenen Oligomeren, dadurch gekennzeichnet, dass man die Umsetzung der 2,4-Dicyanglutarate mit 0,5-1 mol Formaldehyd und/oder Polyoxymethylen in Gegenwart von 0,5-5 mol Wasser jeweils pro mol der 2,4-Dicyandiglutarate bei pH-Werten zwischen 3 und 7 durchführt.

Bei der Herstellung von monomeren 2-Cyanacrylaten ist es üblich, Cyanacetate oder 2,4-Dicyanglutarate mit Formaldehyd und/oder Polyoxymethylen zu kondensieren und die so entstandenen Kondensationsprodukte nach Trocknung thermisch zu spalten. Unter Polyoxymethylen werden hier ringförmige und kettenförmige Polymere des Formaldehyds verstanden, z. B. Trioxan und/oder Paraformaldehyd. Dabei galt es bisher als gesicherte Erkenntnis, dass die Kondensationsreaktion basisch zu katalysieren sei. Darüber hinaus war es üblich, das bei der Kondensation aus Formaldehyd entstehende Reaktionswasser während der Umsetzung durch Schleppmittel, wie Benzol oder Xylol, zu entfernen. Die Erfindung bricht mit diesen Vorstellungen. So wird vorgeschlagen, stattdessen bei pH-Werten zwischen 3 und 7 zu kondensieren.

Das erfindungsgemässe Verfahren liefert – bezogen auf die nachfolgende thermische Spaltung – die besten Resultate, wenn in einem pH-Bereich zwischen 3 und 7, vorzugsweise zwischen 4 und 6 gearbeitet wird. Zur Erreichung dieses pH-Wertes kann eine Vielzahl organischer oder anorganischer Säuren zugesetzt werden. Es ist jedoch in jedem Fall bevorzugt, nichtflüchtige Säuren oder saure Salze zu verwenden. So sind insbesondere saure Salze der Phosphorsäure oder der Schwefelsäure geeignet. In vielen Fällen enthalten die eingesetzten 2,4-Dicyanglutarate bereits durch Hydrolyse entstandene Carbonsäuregruppen, so dass Aufschlämmungen der Ausgangsprodukte in Wasser bereits den für die Reaktion erforderlichen pH-Wert aufweisen. In manchen Fällen ist der Säuregehalt so gross, dass zum Erreichen des geforderten pH-Bereichs Neutralisation erforderlich ist. Geeignet sind nicht flüchtige Neutralisationsmittel, welche unter Bedingungen der thermischen Spaltung keine Nebenprodukte erwarten lassen. So sind insbesondere Hydroxide der Alkali- und Erdalkalimetalle geeignet. Es können auch im pH-Bereich 3-7 wirksame Puffergemische eingesetzt werden wie beispielsweise Mischungen von Hydrogenphosphaten und Dihydrogenphosphaten.

Zur Durchführung des erfindungsgemässen Verfahrens muss auf Einhaltung einer bestimmten Wassermenge geachtet werden. Dabei sollte die Wassermenge 0,5-5 mol Wasser pro mol 2,4-Dicyanglutarat betragen. Besonders günstige Ergebnisse werden erhalten, wenn zwischen 0,8 und 1,2 mol Wasser pro mol 2,4-Dicyanglutarat eingesetzt werden.

In den genannten Wassermengen sind die 2,4-Dicyanglutarate nicht oder nicht vollständig löslich. Vielmehr entstehen unter Reaktionsbedingungen Dispersionen mit emulsionsartigem Aussehen und, was verfahrensmässig erwünscht ist, geringer Viskosität.

Nach dem erfindungsgemässen Verfahren werden im Kondensationsschritt pro mol 2,4-Dicyanglutarat 0,5-1 mol Formaldehyd und/oder Polyoxymethylen eingesetzt. Es ist möglich, Formaldehyd als wässerige Lösung einzusetzen. Nach einer bevorzugten Ausführungsform wird statt Formaldehyd Polyoxymethylen verwendet. Auch Mischungen, d. h. zum Beispiel unstabilisierte Formaldehydlösungen mit einem gewissen Anteil an Polyoxymethylen insbesondere Paraformaldehyd können verwendet werden. In allen Fällen beträgt das bevorzugte Einsatzmolverhältnis 0,60-0,85 mol Formaldehyd oder dessen Derivat pro mol 2,4-Dicyanglutarat.

Zur Durchführung des erfindungsgemässen Verfahrens wird zunächst das 2,4-Dicyanglutarat mit Wasser in einem geeigneten Reaktionsgefäss zum Beispiel einem Rührautoklaven gemischt, wobei eine emulsionsartige Dispersion entsteht. Es wird sodann mit Säuren oder falls diese bereits in den Ausgangsstoffen vorhanden sind, mit Neutralisationsmitteln der gewünschte pH-Bereich von 3-7, vorzugsweise von 4-6 eingestellt. Sodann wird mit Formaldehyd und/oder Polyoxymethylen versetzt. Das Reaktionsgefäss wird verschlossen und auf eine Temperatur von 70-140° C, vorzugsweise 100-120° C aufgeheizt, wobei ein Überdruck entsteht. Der Reaktionsansatz wird 15-60 Minuten, vorzugsweise 30-45 Minuten, bei dieser Temperatur unter Druck belassen. Danach wird das Reaktionsgefäss entspannt und das zugesetzte Wasser sowie das Reaktionswasser von dem entstandenen Kondensationsprodukt destillativ abgetrennt.

Das Kondensationsprodukt unterscheidet sich nicht wesentlich von dem Kondensationsprodukt aus Cyanacetat und einem Unterschuss von Formaldehyd, welches z. B. nach bekannten Verfahren hergestellt werden kann. Nach der Trocknung, welche vorzugsweise bei 100-120° C im Dampfstrahlvakuum oder im Ölpumpenvakuum (Druck unter 1 mbar) ausgeführt wird, werden die Produkte zu 2-Cyanacrylaten gespalten. Hierbei kann beispielsweise nach dem Verfahren der deutschen Auslegeschrift 1 258 405 gearbeitet werden.

Nach dem erfindungsgemässen Verfahren können 2,4-Dicyanglutarate unterschiedlicher Reinheit sowie auch Ester der 2,4-Dicyanglutarsäure mit unterschiedlichsten Alkoholen verwendet werden. So sind z. B. chemisch reine oder fast reine 2,4-Dicyanglutarate verwendbar, welche nach dem Verfahren von H. Hellmann u. K. Stegmüller, beschrieben in Chem. Berichte *90*, 535 (1957) hergestellt worden sind. Es ist jedoch bevorzugt, die bei der Herstellung von 2-Cyanacrylaten als Nebenprodukte anfallenden 2,4-Dicyanglutarate zu verwenden. Derartige Nebenprodukte weisen typischerweise einen Gehalt von etwa 50-85 Gew.-% 2,4-Dicyanglutarat auf. Sie können weiterhin oligomere Cyanacrylate sowie Nebenprodukte enthalten.

Nach dem erfindungsgemässen Verfahren werden bevorzugt symmetrische Ester der 2,4-Dicyanglutarsäure verarbeitet. Dies sind Ester, bei denen beide Carboxylgruppen mit demselben Alkohol umgesetzt worden sind. Geeignete Ester leiten sich insbesondere von folgenden Alkoholen ab: Methanol, Propanol, Butanol, Pentanol, Hexanol sowie den Isomeren der letztgenannten, Cyclohexanol, Benzylalkohol, Monoether des Ethylenglykols oder des Propylenglykols, 2-Ethylhexanol sowie von geradkettigen, verzweigten oder cyclischen Monoalkoholen oder Etheralkoholen mit mehr als 8 und weniger als 20 C-Atomen. Darüber hinaus ist das erfindungsgemässe Verfahren für Phenolester besonders geeignet.

Die Vorteile des erfindungsgemässen Verfahrens liegen in der erhöhten Ausbeute an 2-Cyanacrylaten im Vergleich zum Stand der Technik im Verzicht auf die Verwendung von organischen Lösungsmitteln, in der geringen Viskosität des Reaktionsmediums, welches nur geringen Aufwand von Rührenergie erfordert sowie in der hohen Reinheit der letztendlich erhaltenen 2-Cyanacrylate.

Die folgenden Beispiele sollen das Verfahren erläutern:

*Beispiel 1*

In einem 400 l Rührbehälter wurden 102 kg (ungefähr 400 mol) 2,4-Dicyanglutarsäurediethylester (ca. 94%ig) mit 20 kg Wasser vermischt und 8,5 kg (261 mol) Polyoxymethylen (92%ig) zugegeben. Zur Einstellung eines pH-Werts zwischen 4 und 6 wurden 383 g einer 32%igen NaOH-Lösung (entspricht 3,08 mol) zugesetzt. Die Reaktion wurde unter Rühren bei einer Temperatur zwischen 80 und 130° C im geschlossenen Behälter unter Eigendruck durchgeführt. Die Reaktionszeit betrug 45 Minuten. Nach Abdestillieren des Wassers und Entwässern im Dampfstrahlvakuum wurde eine Kondensationsprodukt erhalten, welches der thermischen Spaltung unterzogen wurde. Destillation des bei der Spaltung entstandenen Rohproduktes ergab 48,6 kg (389 mol) 2-Cyanacrylsäureethylester. Die Ausbeute betrug somit 51 Gew.-%, bezogen auf das eingesetzte Dicyanglutarat.

*Beispiel 2*

In einem 400 l Rührbehälter wurden 130 kg Destillationsrückstand aus der 2-Cyanacrylatproduktion mit einem Gehalt von 75,1 Gew.-% 2,4-Dicyanglutarsäurediethylester (410 mol) mit 21,3 kg Wasser vermischt und in Gegenwart von 804,6 g 32%iger NaOH-Lösung, welche zur Einstellung eines pH-Werts zwischen 4 und 6 benötigt wurde, mit 8,83 kg 92%igem Polyoxymethylen (271 mol) zur Reaktion gebracht. Reaktion und Aufarbeitung wurden wie in Beispiel 1 beschrieben durchgeführt. Es wurden 58,7 kg reiner 2-Cyanacrylsäureethylester erhalten. Dies entspricht einer Ausbeute von 60,1 Gew.-% bezogen auf 100%iges 2,4-Dicyanglutarat.

Die nach Beispiel 1 und 2 hergestellten Monomeren entsprachen den bei Reaktionsklebstoffen üblichen Qualitätsanforderungen.

*Vergleichsbeispiel*

494,0 g (2,074 mol) 2,4-Dicyanglutarsäurediethylester wurden ohne Wasserzusatz mit 44,8 g Polyoxymethylen (92%ig; 1,372 mol) und 3 g einer 30%igen Lösung von Natriummethylat in Methanol (0,0167 mol) zur Reaktion gebracht. Die verwendete Menge an Natriummethylat entsprach einem Überschuss von 1,25 millimol pro mol Dicyanglutarat. Die Reaktion wurde bei einer Temperatur von 100-130° C während 45 Minuten durchgeführt. Anschliessend wurde im Ölpumpenvakuum entwässert. Die thermische Spaltung ergab nach Destillation der Spaltprodukte 154,5 g (1,236 mol) 2-Cyanacrylsäureethylester. Die Ausbeute betrug somit 31,3 Gew.-% bezogen auf 2,4-Dicyanglutarat.

## Patentansprüche

1. Verfahren zur Herstellung von monomeren 2-Cyanacrylaten durch Umsetzung von 2,4-Dicyanglutaraten mit Formaldehyd und/oder Polyoxymethylen und anschliessender thermischer Spaltung der entstandenen Oligomeren, dadurch gekennzeichnet, dass man die Umsetzung der 2,4-Dicyanglutarate mit 0,5-1 mol Formaldehyd und/oder Polyoxymethylen in Gegenwart von 0,5-5 mol Wasser jeweils pro mol der 2,4-Dicyanglutarate bei pH-Werten zwischen 3 und 7 durchführt.

2. Verfahren nach Anspruch 1, dadurch gekennzeichnet, dass man die Umsetzung in Gegenwart schwerflüchtiger, insbesondere anorganischer Säuren und/oder deren sauren Salzen durchführt.

3. Verfahren nach den Ansprüchen 1 und 2, dadurch gekennzeichnet, dass man als 2,4-Dicyanglutarat Nebenprodukte aus der 2-Cyanacrylat-Synthese einsetzt, die 50-85 Gew.-% 2,4-Dicyanglutarat enthalten.

4. Verfahren nach den Ansprüchen 1 bis 3, dadurch gekennzeichnet, dass man pro mol 2,4-Dicyanglutarat 0,8-1,2 mol Wasser verwendet.

5. Verfahren nach den Ansprüchen 1 bis 4, dadurch gekennzeichnet, dass man pro mol 2,4-Dicyanglutarat 0,60-0,85 mol Formaldehyd und/oder Polyoxymethylen einsetzt.

6. Verfahren nach den Ansprüchen 1 bis 5, dadurch gekennzeichnet, dass man die Kondensation bei einer Temperatur von 70-140° C, vorzugsweise zwischen 100-120° C im geschlossenen Reaktionsgefäss unter Druck durchführt.

## Claims

1. A process for the production of monomeric 2-cyanacrylates by reaction of 2,4-dicyanoglutarates with formaldehyde and/or polyoxymethylene and subsequent thermal cleavage of the oligomers formed, characterized in that the reaction of the 2,4-dicyanoglutarates is carried out with from 0.5 to 1 mole of formaldehyde and/or polyoxymethylene in the presence of from 0.5 to 5 moles of water per mole of the 2,4-dicyanoglutarates at pH-values of from 3 to 7.

2. A process as claimed in Claim 1, characterized in that the reaction is carried out in the presence of substantially involatile, in particular inorganic acids and/or acidic salts thereof.

3. A process as claimed in Claims 1 and 2, characterized in that secondary products from the synthesis of 2-cyanoacrylates containing from 50 to 85% by weight of 2,4-dicyanoglutarate are used as the 2,4-dicyanoglutarate.

4. A process as claimed in Claims 1 to 4, characterized in that from 0.8 to 1.2 moles of water are used per mole of 2,4-dicyanoglutarate.

5. A process as claimed in Claims 1 to 4, characterized in that from 0.60 to 0.85 moles of formaldehyde and/or polyoxymethylene is used per mole of 2,4-dicyanoglutarate.

6. A process as claimed in Claims 1 to 5, characterized in that the condensation is carried out at a temperature of from 70 to 140° C and preferably at a temperature of from 100 to 120° C under pressure in a closed reaction vessel.

## Revendications

1. Procédé de fabrication de 2-cyanacrylates monomères par réaction de 2,4-dicyanoglutarates avec du formaldéhyde et/ou du polyoxyméthylène et clivage thermique consécutif des oligomères obtenus, caractérisé en ce qu'on exécute la réaction des 2,4-dicyanoglutarates avec 0,5 à 1 mole de formaldéhyde et/ou de polyoxyméthylène en présence de 0,5 à 5 moles d'eau, chaque fois par mole des 2,4-dicyanoglutarates, à des valeurs de pH comprises entre 3 et 7.

2. Procédé selon la revendication 1, caractérisé en ce qu'on exécute la réaction en présence d'acides difficilement volatils, en particulier minéraux, et/ou de leurs sels acides.

3. Procédé selon les revendications 1 et 2, caractérisé en ce qu'on utilise comme 2,4-dicyanoglutarate les sous-produits de la synthèse du 2-cyanacrylate qui contiennent 50 à 85% en poids de 2,4-dicyanoglutarate.

4. Procédé selon les revendications 1 à 3, caractérisé en ce qu'on utilise par mole de 2,4-dicyanoglutarate 0,8 à 1,2 mole d'eau.

5. Procédé selon les revendications 1 à 4, caractérisé en ce qu'on utilise par mole de 2,4-dicyanoglutarate 0,60 à 0,85 mole de formaldéhyde et/ou de polyoxyméthylène.

6. Procédé selon les revendications 1 à 5, caractérisé en ce qu'on exécute la condensation à une température de 70 à 140° C, de préférence entre 100 et 120° C, sous pression dans un récipient de réaction fermé.